# EUROPEAN PATENT SPECIFICATION

(11) **EP 3 700 507 B1**
(45) Date of publication and mention of the grant of the patent: **20.10.2021**
(21) Application number: 18804132.1
(22) Date of filing: 24.10.2018
(51) Int. Cl.: A61K 9/08, A61K 47/40, A61K 31/202, A61P 27/02

(54) **STABLE AQUEOUS SOLUTIONS COMPRISING PUFAS**
STABILE WÄSSRIGE LÖSUNGEN MIT PUFAS
SOLUTIONS AQUEUSES STABLES COMPRENANT DES AGPI

(30) Priority: 24.10.2017 IT 201700120375
(43) Date of publication of application: 02.09.2020
(73) Proprietor: Medivis S.r.l., 95030 Tremestieri Etneo, Catania (IT)
(72) Inventor: ALEO, Danilo, 95127 Catania (IT); MANGIAFICO, Sergio, 95127 Catania (IT); SAITA, Maria Grazia, 95127 Catania (IT); MELILLI, Barbara, 95127 Catania (IT); CRO, Melina, 95127 Catania (IT); MANGIAFICO, Sebastiano, 95127 Catania (IT)
(74) Representative: Croce, Valeria
(86) International application number: PCT/IB2018/058304
(87) International publication number: WO 2019/082102

(56) References cited:
- EP-A1- 2 921 171
- WO-A1-2009/136179
- US-A- 4 438 106
- US-A- 5 977 180
- US-A1- 2003 203 034

## Description

The present invention relates to a composition for ophthalmic use comprising one or more polyunsaturated fatty acids with free carboxylic function (PUFAs FFA) in aqueous solution with methyl-β-cyclodextrins (Met-β-CD), wherein said PUFAs are selected from the group comprising omega-3 and omega-6 polyunsaturated fatty acids, said polyunsaturated fatty acids having an aliphatic chain of from 16 to 24 carbon atoms.

The above composition for use in the treatment of ocular pathologies selected from the group of inflammatory diseases affecting the ocular surface is a further object of the invention.

The invention is defined by the claims. Any subject-matter falling outside the scope of the claims is provided for information purposes only.

### Prior art

The use of polyunsaturated fatty acids (PUFAs) in pharmaceutical compositions is strongly limited by the poor solubility in water and by the poor chemical stability of these particular fatty acids. EP 2 921 171 A1, for instance, discloses stable watersoluble compositions for ophthalmic use comprising one or more N-acylethanolamines in the form of an inclusion complex in methyl-β-cyclodextrin. The N-acylethanolamine is selected from N-palmitoylethanolamine, N-oleylethanolamine, N-stearoylethanolamine, N-α-linolenoylethanolamine, N-y-linolenoylethanolamine, N-linoleylethanolamine, N-eicosapentaenoylethanolamine and N-docoesaenoylethanolamine.

The difficulties related to the poor water solubility of PUFAs have been addressed by the prior art using heterogeneous systems such as emulsions, or dispersed oils systems in semi-solid aqueous media, obtained with hydrophilic polyacrylic polymers and structures like Noveon, a high molecular weight acrylic acid polymer cross-linked with divinyl glycol, and Carbopol, a vinyl polymer.

Lipid emulsions, which have been used for some time for parenteral applications, have been studied in more recent times to formulate ophthalmic preparations of different lipophilic active ingredients and improve the ocular bioavailability thereof.

EP0391369 describes the use of lipid emulsions, in particular oil-in-water emulsions, as a carrier for lipophilic active ingredients. However, this method, when applied to formulations containing PUFAs as active ingredients, has given unsatisfactory results, since the resulting emulsion is unstable from the physical point of view, tending to evolve to phase and chemical separation, as the eicosapentaenoic acid (EPA) and gamma-linolenic acid (GLA) titer is significantly reduced already after the first month of storage at 25 °C (EPA 96% and GLA 95%) and after four months at 4 °C, the content of EPA drops to 85% and that of GLA to 90%, most likely due to oxidation.

WO2006/007510 describes ophthalmic compositions for topical use which include omega-3 and omega-6 fatty acids. The omega-3 and -6 fatty acids are emulsified with surfactants, such as polyhexylated fatty acids and sorbitan esters, or polysorbates such as "Tween", and polyethoxylated methylglucosides such as "Glucam". Formulations based on EPA, docosahexaenoic acid (DHA) and GLA obtained in emulsion according to said method, however, undergo physical and chemical instability. WO2010/106571 discloses formulations for ophthalmic use which include PUFAs and vitamin E acetate. Said formulations are stable for 12 months. After 12 months at 4 °C and at 25 °C, the PUFAs weakly degrade, following a linear trend.

WO2016/162894 discloses formulations for ophthalmic use which include PUFAs. Studies of prolonged stability at 24 months at 25 °C show rapid degradation, with a polynomial trend.

WO2014/035450 discloses topical ophthalmic preparations based on omega-3 fatty acids suspended in an aqueous gel, wherein the gel consists of a weakly cross-linked carboxylic polymer with an adequate concentration of components in the form of ionic salts, such as to have an elastic behavior typical of gels, i.e. with an elastic modulus (G') greater than the viscous module (G") (in particular G' > 5 G"). The ophthalmic preparation, mixed in the ratio of between 2:1 and 5:1 with the tear fluid, rich in salts, would form a mixture having a module G" > G', thus changing from the typical elastic behavior of gels to a liquid behavior of suitable viscosity capable of being tolerated by the eye. This solution is not applicable to pathological situations leading to lacrimal dysfunctions such as Sjögren's syndrome, dry eye syndrome and in case of inflammatory states of the ocular surface, when the tear fluid is much less than 6 µl (Scherz W. et al., Tear Volume in Normal Eyes and Keratoconjunctivitis sicca, Arbrecht v. Graefes Arch. Klin. Exp. Ophthal. 192, 141-150, 1974). On the other hand, the administration of ocular drops almost always exceeds 30 µl, consequently the volumes involved never produce the ratio of tear fluid/eye drops volumes sufficient to have the Gel-Sol transition. This lack of transition from the elastic gel to viscous liquid gives the patient the sensation of sticky eyelids and distorted vision. The preparation from the physical point of view is stable for a few weeks and the chemical stability data of the omega-3 contained therein is not reported.

There is therefore a strong need to have compositions based on PUFAs that are stable from a physical and chemical point of view and with good ocular tolerance.

### Detailed description of the invention

The object of the present invention is an aqueous solution which comprises PUFAs and Methyl-β-Cyclodextrins as solubilizing agents. Said composition is chemically and physically stable without having to resort to the use of heterogeneous emulsions or suspensions in hydrogels. Said composition is particularly advantageous, since it is particularly stable, whereas said PUFAs are polyunsaturated fatty acids having the free carboxylic function (PUFAs FFA) and not esterified.

Said PUFAs are omega-3 and omega-6 which have an aliphatic chain of from 16 to 24 carbon atoms. Alternatively, said aliphatic chain is 18-24 carbon atoms.

Said composition is an aqueous solution which comprises PUFAs in a concentration of between 0.01% w/w and 0.10% w/w and Methyl-β-Cyclodextrin between 1.0 and 10% w/w. Preferably, said PUFAs are in a concentration of between 0.02 and 0.07% w/w, or between 0.03 and 0.06% w/w and said Met-β-CD are between 2.0 and 8% w/w.

In the present invention, said PUFAs are PUFAs FFA. Even more preferably, said PUFAs are selected from the group consisting of: EPA, DHA, GLA.

The pH of said solutions is about the physiological pH and said compositions are buffered, for example with phosphate, borate or citrate buffer.

Said compositions are particularly stable over time from the physical-chemical point of view.

In an embodiment, one aspect of the present invention is a formulation comprising, in addition to PUFAs and Met-β-CD, viscous polymers, for example Carbopol, sodium hyaluronate, hydroxypropylmethyl cellulose (HPMC), or hydroxypropyl guar (HPG).

In a further embodiment, said formulation comprises osmotizing agents such as mannitol, glycerol and/or trehalose.

Said formulation optionally comprises sequestering agents, such as EDTA.

In a further embodiment, said formulation comprises emollients having anti-inflammatory activity for the ocular surface, such as for example Adelmidrol.

In one embodiment, said formulation comprises preservatives, for example ascorbyl glycoside, or antimicrobials.

Further active ingredients known to the man skilled in the art are co-formulated with the composition according to the present invention, in alternative embodiments.

The composition according to the present invention is advantageously used in eye drops and ointments for ophthalmic use.

A further aspect of the present invention is said composition for use in the treatment of ocular pathologies selected from the group of inflammatory diseases affecting the ocular surface such as, for example, blepharitis, conjunctivitis, keratoconjunctivitis, or anterior uveitis.

The following examples have the purpose of better illustrating the invention, not wanting to be in any way limiting thereof.

### Examples 1-5: compositions comprising fatty acids with a free carboxylic function EPA FFA and DHA FFA and Methyl-β-cyclodextrin (Met-β-CD)

Table 1 shows exemplary compositions according to the present invention.

**Table 1**

| **Composition** | **Example 1 % w/w** | **Example 2 % w/w** | **Example 3 % w/w** | **Example 4 % w/w** | **Example 5 % w/w** |
|---|---|---|---|---|---|
| DHA FFA | 0.03 | 0.04 | | | 0.03 |
| EPA FFA | | | 0.05 | 0.03 | 0.03 |
| Met-β-CD | 3.0 | 3.0 | 5.0 | 3.0 | 6.0 |
| Na₂HPO₄ | 0.1 | 0.1 | 0.1 | 0.1 | 0.1 |
| NaH₂PO₄*2H₂O | 0.06 | 0.06 | 0.06 | 0.06 | 0.06 |
| Glycerol | 0.9 | 0.9 | 0.9 | 0.9 | 0.9 |
| Mannitol | 1.0 | 1.0 | 1.0 | 1.0 | 1.0 |
| EDTA-Na₂*2H₂O | 0.02 | 0.02 | 0.02 | 0.02 | 0.02 |
| NaCl | 0.2 | 0.2 | 0.2 | 0.2 | 0.2 |
| Purified water | Up to 100 | Up to 100 | Up to 100 | Up to 100 | Up to 100 |

The compositions described are particularly stable over time. By way of example, tables 2 and 3 show the stability data at 25 °C and 40 °C of DHA FFA in the formulation of example 1 and EPA FFA in the formulation of example 3. Said FFA PUFAs maintain a titer of at least 95% after 24 months of storage at room temperature. Comparable data are obtained for the compositions referred to in Examples 2, 4 and 5.

**Table 2**

| **Composition** | **Example 1 % w/w** | **Stability** | | **0 months** | **3 months** | **6 months** | **12 months** | **24 months** |
|---|---|---|---|---|---|---|---|---|
| DHA FFA | 0.03 | **25 °C** | % DHA FFA | 100 | 100 | 99 | 98 | 95 |
| EPA FFA | -- | | | | | | | |
| Met-β-CD | 3.0 | | | | | | | |
| Na₂HPO₄ | 0.1 | | pH | 6.8 | 6.8 | 6.8 | 6.8 | 6.7 |
| NaH₂PO₄*2H₂O | 0.06 | | | | | | | |
| Glycerol | 0.9 | **40** °C | % DHA FFA | 100 | 98 | 95 | -- | -- |
| Mannitol | 1.0 | | | | | | | |
| EDTA-Na₂*2H₂O | 0.02 | | | | | | | |
| NaCl | 0.2 | | pH | 6.8 | 6.8 | 6.7 | -- | -- |
| Purified water | Up to 100 | | | | | | | |

**Table 3**

| **Composition** | **Example 3 % w/w** | **Stability** | | **0 months** | **3 mont hs** | **6 months** | **12 months** | **24 months** |
|---|---|---|---|---|---|---|---|---|
| DHA FFA | -- | **25°C** | % EPA FFA | 100 | 99 | 99 | 98 | 96 |
| EPA FFA | 0.05 | | | | | | | |
| Met-β-CD | 5.0 | | | | | | | |
| Na₂HPO₄ | 0.1 | | pH | 6.9 | 6.9 | 6.8 | 6.8 | 6.8 |
| NaH₂PO₄*₂H2O | 0.06 | | | | | | | |
| Glycerol | 0.9 | **40** °C | % EPA FFA | 100 | 97 | 95 | -- | -- |
| Mannitol | 1.0 | | | | | | | |
| EDTA-Na₂*2H₂O | 0.02 | | | | | | | |
| NaCl | 0.2 | | pH | 6.9 | 6.8 | 6.7 | -- | -- |
| Purified water | Up to 100 | | | | | | | |

### Examples 6-10: esterified fatty acids EPA EE and DHA EE and Met-β-CD (comparative)

Table 4 shows compositions for comparative purposes.

**Table 4**

| **Composition** | **Example 6 % w/w** | **Example 7 % w/w** | **Example 8 % w/w** | **Example 9 % w/w** | **Example 10 % w/w** |
|---|---|---|---|---|---|
| DHA EE | 0.03 | 0.04 | -- | -- | 0.03 |
| EPA EE | -- | -- | 0.05 | 0.03 | 0.03 |
| Met-β-CD | 3.0 | 5.0 | 6.0 | 4.0 | 6.0 |
| Na₂HPO₄ | 0.1 | 0.1 | 0.1 | 0.1 | 0.1 |
| NaH₂PO₄*2H₂O | 0.06 | 0.06 | 0.06 | 0.06 | 0.06 |
| Glycerol | 0.9 | 0.9 | 0.9 | 0.9 | 0.9 |
| Mannitol | 1.0 | 1.0 | 1.0 | 1.0 | 1.0 |
| EDTA-Na₂*2H₂O | 0.02 | 0.02 | 0.02 | 0.02 | 0.02 |
| NaCl | 0.2 | 0.2 | 0.2 | 0.2 | 0.2 |
| Purified water | Up to 100 | Up to 100 | Up to 100 | Up to 100 | Up to 100 |

Table 5 shows the stability data of the composition as per example 6. DHA EE is almost completely degraded after 3 months.

**Table 5**

| **Composition** | **Example 6 % w/w** | **Stability** | | **0 months** | **3 months** |
|---|---|---|---|---|---|
| DHA EE | 0.03 | **25 °C** | % DHA EE | 100 | 8 |
| EPA EE | -- | | | | |
| Met-β-CD | 3.0 | | | | |
| Na₂HPO₄ | 0.1 | | pH | 6.8 | 6.7 |
| NaH₂PO₄*2H₂O | 0.06 | | | | |
| Glycerol | 0.9 | **40** °C | % DHA EE | 100 | 0.5 |
| Mannitol | 1.0 | | | | |
| EDTA-Na₂*2H₂O | 0.02 | | | | |
| NaCl | 0.2 | | pH | 6.8 | 6.7 |
| Purified water | Up to 100 | | | | |

### Examples 11-15: fatty acids with free carboxylic function EPA FFA and DHA FFA and HP-β-CD cyclodextrin, HP-γ-CD, γ-CD, SEB-β-CD (comparative)

To highlight the surprising advantage observed with Met-β-CD, table 6 shows examples of fatty acid formulations with free carboxylic function in the presence of hydroxypropyl-β-cyclodextrin (HP-β-CD), hydroxypropyl-γ-cyclodextrin (HP-γ-CD), γ-cyclodextrin (γ-CD) or sulfobutyl ether β-cyclodextrin (SBE-β-CD). The reported concentrations of β cyclodextrins of examples 11, 14 and 15 are the minimum ones for oil solubilization.

**Table 6**

| **Composition** | **Example 11 % w/w** | **Example 12 % w/w** | **Example 13 % w/w** | **Example 14 % w/w** | **Example 15 % w/w** |
|---|---|---|---|---|---|
| DHA FFA | 0.03 | 0.04 | | | 0.03 |
| EPA FFA | | | 0.05 | 0.03 | 0.03 |
| HP-β-CD | 5 | | | | 7 |
| HP-γ-CD | | 5 | | | |
| γ-CD | | | 5 | | |
| SBE β-CD | | | | 6 | |
| Na₂HPO₄ | 0.1 | 0.1 | 0.1 | 0.1 | 0.1 |
| NaH₂PO₄*2H₂O | 0.06 | 0.06 | 0.06 | 0.06 | 0.06 |
| Glycerol | 0.9 | 0.9 | 0.9 | 0.3 | 0.9 |
| Mannitol | 1.0 | 1.0 | 1.0 | 1.0 | 1.0 |
| EDTA-Na₂*2H₂O | 0.02 | 0.02 | 0.02 | 0.02 | 0.02 |
| NaCl | 0.2 | 0.2 | 0.2 | -- | 0.2 |
| Purified water | Up to 100 | Up to 100 | Up to 100 | Up to 100 | Up to 100 |

Formulations in the presence of cyclodextrins other than Met-β-CD do not lead to stable formulations. Table 7 shows the stability of the formulation of example 11, where said composition differs from that according to the present invention and described in example 1 only in the different cyclodextrin.

**Table 7**

| **Composition** | **Example 11 % w/w** | **Stability** | | **0 months** | **3 months** |
|---|---|---|---|---|---|
| DHA FFA | 0.03 | **25 °C** | % DHA FFA | 100 | 38 |
| EPA FFA | -- | | | | |
| HP-β-CD | 5.0 | | | | |
| Na₂HPO₄ | 0.1 | | pH | 6.8 | 6.8 |
| NaH₂PO₄*2H₂O | 0.06 | | | | |
| Glycerol | 0.9 | **40** °C | % DHA FFA | 100 | 11 |
| Mannitol | 1.0 | | | | |
| EDTA-Na₂*2H₂O | 0.02 | | | | |
| NaCl | 0.2 | | pH | 6.8 | 6.7 |
| Purified water | Up to 100 | | | | |

Results similar to those obtained with HP-β-CD were obtained with sulfobutyl ether β-cyclodextrins (not shown) . The formulations with γ-CDs instead gave rise to insoluble aggregates not useful for the purpose of obtaining homogeneous aqueous solutions of PUFAs, both in the form of free acid and of ethyl ester.

### Examples 16-26: formulations comprising fatty acids with a free carboxylic function EPA FFA and DHA FFA and Met-β-CD

Table 8 shows stable formulations according to the present invention containing polyunsaturated fatty acids with free carboxylic function and Met-β-CD in the presence of viscosizing polymers such as carbopol, sodium hyaluronate, hydroxypropylmethyl cellulose (HPMC) and hydroxypropyl guar (HPG) and osmotizing agents such as mannitol, glycerol and trehalose.

**Table 8**

| **Composition** | **Example 16 % w/w** | **Example 17 % w/w** | **Example 18 % w/w** | **Example 19 % w/w** | **Example 20 % w/w** | **Example 21 % w/w** |
|---|---|---|---|---|---|---|
| PUFAs (FFA) | 0.03 | 0.03 | 0.03 | 0.03 | 0.03 | 0.03 |
| Carbopol | | 0.2 | | | 0.2 | |
| Sodium hyaluronate | 0.15 | | | 0.15 | | |
| HPMC | | | 0.5 | | | |
| HPG | | | | | | 0.5 |
| Met-β-CD | 3.0 | 2.0 | 3.0 | 2.0 | 6.0 | 2 |
| Na₂HPO₄ | 0.1 | 0.1 | 0.1 | 0.1 | 0.1 | |
| NaH₂PO₄*2H₂O | 0.06 | 0.06 | 0.06 | 0.06 | 0.06 | |
| Glycerol | 0.9 | 0.5 | 0.9 | 0.9 | 0.9 | 0.7 |
| Mannitol | 1.0 | 1.0 | | 1.0 | 1.0 | |
| Trealose*2H₂O | 0.5 | 1.0 | 2.0 | | | 2 |
| EDTA-Na₂*2H₂O | 0.02 | 0.02 | 0.02 | 0.02 | 0.02 | 0.02 |
| NaCl | 0.2 | 0.2 | 0.2 | 0.2 | 0.2 | 0.1 |
| Purified water | Up to 100 | Up to 100 | Up to 100 | Up to 100 | Up to 100 | Up to 100 |

The formulations of examples 16-21, in a further embodiment, contain other excipients with emollient activity on the ocular surface including adelmidrol, which also has anti-inflammatory activity also known for its control action on the release of mast cell mediators with a pro-inflammatory effect, and preservatives, among which ascorbyl glycoside employed in a percentage of between 0.005% and 0.02%. In further embodiments, said formulations also comprise active ingredients having steroidal anti-inflammatory activity and NSAIDs, including bromfenac, indomethacin, piroxicam, diclofenac, ketorolac, nepafenac and acetylsalicylic acid. Table 9 shows some examples of said embodiments.

The excipients tested in examples 16-26 do not modify the stability of PUFAs which remains comparable to that observed in the PUFAs-FFA compositions in the presence of Met-β-CD.

| **Composition** | **Example 22 % w/w** | **Example 23 % w/w** | **Example 24 % w/w** | **Example 25 % w/w** | **Example 26 % w/w** |
|---|---|---|---|---|---|
| PUFAs (FFA) | 0.03 | 0.015 | 0.03 | 0.015 | 0.03 |
| Adelmidrol | 0.4 | 0.4 | 0.2 | 0.4 | 0.8 |
| Sodium | 0.15 | 0.15 | -- | 0.15 | 0.1 |
| hyaluronate | | | | | |
| HPMC | 0.1 | -- | 0.5 | -- | 0.5 |
| HPG | -- | -- | -- | 0.2 | -- |
| Met-β-CD | 3.0 | 2.0 | 3.0 | 2.0 | 6.0 |
| Na₂HPO₄ | 0.1 | 0.1 | 0.1 | 0.1 | 0.1 |
| NaH₂PO₄*2H₂O | 0.06 | 0.06 | 0.06 | 0.06 | 0.06 |
| Glycerol | 0.9 | 0.9 | 0.9 | 0.9 | 0.9 |
| Mannitol | 1.0 | -- | -- | -- | 1.0 |
| Ascorbyl glucoside | 0.015 | 0.08 | 0.015 | -- | -- |
| EDTA-Na₂*2H₂O | 0.02 | 0.02 | 0.02 | 0.02 | 0.02 |
| NaCl | 0.2 | 0.3 | 0.2 | 0.3 | 0.2 |
| Purified water | Up to 100 | Up to 100 | Up to 100 | Up to 100 | Up to 100 |

## Claims

1. A composition for ophthalmic use comprising one or more polyunsaturated fatty acids with free carboxylic function (PUFAs FFA) selected from the group comprising omega-3 and omega-6 polyunsaturated fatty acids, said polyunsaturated fatty acids having an aliphatic chain of from 16 to 24 carbon atoms, in aqueous solution with methyl-β-cyclodextrins (Met-β-CD) .

2. A composition according to claim 1, wherein said PUFAs FFA have a concentration of between 0.01% w/w and 0.10% w/w and said Met-β-CD have a concentration of between 1.0 and 10% w/w.

3. A composition according to one of claims 1 or 2, wherein said PUFAs FFA have a concentration of between 0.02 and 0.07% w/w, or between 0.03 and 0.06% w/w and said Met-β-CD of between 2.0 and 8% w/w.

4. A composition according to one of claims 1 to 3, wherein said PUFAs are selected from the group consisting of: eicosapentaenoic acid (EPA), docosahexaenoic acid (DHA), gamma-linolenic acid (GLA) .

5. A composition according to one of claims 1 to 4, also comprising one or more agents selected from: osmotizers, pH regulators, preservatives, anti-inflammatory agents.

6. A composition according to one of claims 1 to 5, comprising one or more further active compounds.

7. A composition according to one of claims 1 to 6 for use in the treatment of ocular pathologies selected from the group of inflammatory diseases affecting the ocular surface.

## Patentansprüche

1. Zusammensetzung zur ophthalmischen Verwendung, umfassend eine oder mehrere mehrfach ungesättigte Fettsäuren mit freier Carboxylfunktion (PUFAs FFA), ausgewählt aus der Gruppe, umfassend mehrfach ungesättigte Omega-3- und Omega-6-Fettsäuren, wobei die mehrfach ungesättigten Fettsäuren eine aliphatische Kette von 16 bis 24 Kohlenstoffatomen aufweisen, in wässriger Lösung mit Methyl-β-Cyclodextrinen (Met-β-CD).

2. Zusammensetzung nach Anspruch 1, wobei die PUFAs FFA eine Konzentration zwischen 0,01 Gew.-% und 0,10 Gew.-% aufweisen und das Met-β-CD eine Konzentration zwischen 1,0 und 10 Gew.-% aufweist.

3. Zusammensetzung nach einem der Ansprüche 1 oder 2, wobei die PUFAs FFA eine Konzentration zwischen 0,02 und 0,07 Gew.-% oder zwischen 0,03 und 0,06 Gew.-% aufweisen und das Met-β-CD eine Konzentration zwischen 2,0 und 8 Gew.-% aufweist.

4. Zusammensetzung nach einem der Ansprüche 1 bis 3, wobei die PUFAs ausgewählt sind aus der Gruppe, bestehend aus: Eicosapentaensäure (EPA), Docosahexaensäure (DHA), Gamma-Linolensäure (GLA).

5. Zusammensetzung nach einem der Ansprüche 1 bis 4, außerdem umfassend ein oder mehrere Mittel, ausgewählt aus: Osmotisierungsmitteln, pH-Einstellmitteln, Konservierungsmitteln, entzündungshemmenden Mitteln.

6. Zusammensetzung nach einem der Ansprüche 1 bis 5, umfassend einen oder mehrere weitere Wirkstoffe.

7. Zusammensetzung nach einem der Ansprüche 1 bis 6 zur Verwendung bei der Behandlung von Augenkrankheiten, ausgewählt aus der Gruppe entzündlicher Erkrankungen, die die Augenoberfläche betreffen.

## Revendications

1. Composition pour une utilisation ophtalmique comprenant un ou plusieurs acides gras polyinsaturés ayant une fonction carboxylique libre (AGPI AGL) choisis dans le groupe comprenant les acides gras oméga-3 et oméga-6 polyinsaturés, lesdits acides gras polyinsaturés ayant une chaîne aliphatique ayant de 16 à 24 atomes de carbone, en solution aqueuse avec des méthyl-β-cyclodextrines (Met-β-CD).

2. Composition selon la revendication 1, dans laquelle lesdits AGPI AGL ont une concentration entre 0,01 % p/p et 0,10 % p/p, et lesdits Met-β-CD ont une concentration entre 1,0 et 10 % p/p.

3. Composition selon l'une des revendications 1 ou 2, dans laquelle lesdits AGPI AGL ont une concentration entre 0,02 et 0,07 % p/p, ou entre 0,03 et 0,06 % p/p et lesdits Met-β-CD entre 2,0 et 8 % p/p.

4. Composition selon l'une des revendications 1 à 3, dans laquelle lesdits AGPI sont choisis dans le groupe constitué par: l'acide éicosapentaénoïque (EPA), l'acide docosahexaénoïque (DHA), l'acide gamma-linolénique (GLA).

5. Composition selon l'une des revendications 1 à 4, comprenant aussi un ou plusieurs agents choisis parmi : des agents d'osmotisation, des régulateurs de pH, des conservateurs, des agents anti-inflammatoires.

6. Composition selon l'une des revendications 1 à 5, comprenant un ou plusieurs principes actifs supplémentaires.

7. Composition selon l'une des revendications 1 à 6 pour une utilisation dans le traitement de pathologies oculaires choisies dans le groupe des maladies inflammatoires affectant la surface oculaire.
